# FASCICULE DE BREVET EUROPEEN

(11) **EP 0 663 955 B1**
(45) Date de publication et mention de la délivrance du brevet: **05.12.2001**
(21) Numéro de dépôt: 93920905.2
(22) Date de dépôt: 21.09.1993
(51) Int. Cl.: C12N 15/60, C12N 15/81, C12N 9/88, C12N 1/21, C12N 1/19, C12Q 1/68

(54) **ACIDE NUCLEIQUE CODANT POUR UNE ALPHA-ACETOLACTATE DECARBOXYLASE ET SES APPLICATIONS**
FÜR EINE ALPHA-ACETOLACTAT-DECARBOXYLASE KODIERENDE NUKLEINSÄURE UND IHRE ANWENDUNGEN
NUCLEIC ACID CODING FOR AN ALPHA-ACETOLACTATE DECARBOXYLASE AND APPLICATIONS

(30) Priorité: 25.09.1992 FR 9211471
(43) Date de publication de la demande: 26.07.1995
(73) Titulaire: INSTITUT NATIONAL DE LA RECHERCHE AGRONOMIQUE, F-75341 Paris Cédex 07 (FR)
(72) Inventeur: EHRLICH, Stanislav, F-75013 Paris (FR); GODON, Jean-Jacques, F-77140 Saint-Pierre-de-Nemours (FR); RENAULT, Pierre, F-78180 Montigny-le-Bretonneux (FR)
(74) Mandataire: Pernez, Helga
(86) Numéro de dépôt international: FR9300909
(87) Numéro de publication internationale: WO9408019

(56) Documents cités:
- EP-A- 0 228 009
- EP-A- 0 500 188
- US-A- 5 108 925
- APPLIED AND ENVIRONMENTAL MICROBIOLOGY vol. 54, no. 7 , Juillet 1988 pages 1889 - 1891 DETLEF GOELING ET AL 'Cloning and expression of an alpha-Acetolactate Decarboxylase gene fron Streptococcus lactis subsp. diacetylactis in Escherichia coli'
- JOURNAL OF MOLECULAR BIOLOGY vol. 196 , 1987 pages 39 - 48 PH. NOIROT ET AL 'Plasmid Replication stimulates DNA recombination in Bacillus subtilis' cité dans la demande
- K. BLOMQVIST ET AL.: "Characterization of the Genes of the 2,3-Butanediol Operons from Klebsiella terrigena and Enterobacter aerogenes", JOURNAL OF BACTERIOLOGY, , Mars 1993, Vol. 175, no. 5, pages 1392 à 1404

## Description

L'invention concerne un acide nucléique codant pour un polypeptide possédant une activité α-acétolactate décarboxylase ainsi que les vecteurs contenant cet acide nucléique et l'utilisation de ces derniers pour transformer des microorganismes chez lesquels la dégradation de l'α-acétolactate en acétoine sera favorisée. L'invention concerne également les applications de cet acide nucléique pour la conception de nouveaux modes d'inactivation du gène codant pour l'α-acétolactate décarboxylase chez les bactéries de type *L. lactis*, en vue de favoriser chez ces dernières la production de diacétyle.

L'α-acétolactate est un produit naturel du métabolisme des microorganismes. Selon la nature du milieu, l'α-acétolactate peut se dégrader spontanément en un composé aromatique, le diacétyle, ou sous l'action d'une enzyme, l'α-acétolactate décarboxylase, se transformer en acétoine, qui est un composé non aromatique.

Le diacétyle est un arôme recherché dans la production de produits alimentaires comme le beurre, la crème fraîche et certains fromages. A l'inverse, cet arôme est indésirable dans la production d'autres produits alimentaires comme la bière.

L'α-acétolactate est aussi un intermédiaire de la voie de synthèse des acides aminés branchés que sont la leucine, l'isoleucine, et la valine. Cette voie a été très largement étudiée chez les bactéries, les champignons et les plantes (1, 2, 3, 4).

Le brevet américain US No. 5 108 925 revendique un procédé de production accélérée de bière, par le brassage de la bière avec une souche modifiée de Saccharomyces cerevisiae ayant intégré dans son génome une séquence nucléotidique codant pour une α-acétolactate décarboxylase issue des souches *Aerobacter aérogènes* et *Enterobacter aérogènes.*

La figure 3 de ce brevet divulgue une séquence nucléotidique correspondant au gène codant pour cette α-acétolactate décarboxylase issue des souches Aérobacter aérogènes ainsi que la séquence d'acides aminés déduite de la première. Toutefois, les séquences de l'ensemble des gènes impliqués dans cette voie chez un même microorganisme n'ont jamais été rapportées. La régulation de l'expression des gènes impliqués dans la voie de synthèse des acides aminés branchés est complexe en raison des étapes communes pour la synthèse de ces trois acides aminés et cette voie est souvent présentée comme un modèle d'études d'organisation et de régulation.

La synthèse des acides aminés branchés, la leucine, l'isoleucine et la valine chez *L. lactis subsp. lactis* fait intervenir neuf enzymes, dont quatre sont communes à la synthèse des trois acides aminés. La synthèse de la valine fait intervenir uniquement ces quatre enzymes, alors que la synthèse de l'isoleucine et de la leucine fait intervenir respectivement une et quatre enzymes spécifiques supplémentaires. En outre, ce système est compliqué par le fait que l'excès de l'un des trois acides aminés peut interférer sur la synthèses des autres acides aminés. Chez *L. lactis subsp. lactis* le gène codant pour l'α-acétolactate décarboxylase, dénommé gène aldB, est proche des gènes impliqués dans la voie de synthèse des acides aminés branchés et est transcrit soit en même temps que ceux-ci et alors réguler par le taux d'isoleucine, soit indépendamment et alors sous le contrôle d'un gène activateur qui lui est contigu, dénommé gène aldR. Le produit codé par le gène aldB est responsable de la sensibilité des souches de *L. lactis subsp. lactis* à la leucine; en présence de leucine, l'α-acétolactate est détourné de la voie de synthèse de la valine pour être transformé en acétoine.

Chez *E. coli,* les gènes codant pour ces enzymes sont dispersés et contrôlés indépendamment. Dans cette bactérie la plupart des gènes sont assemblés en trois unités contrôlés par atténuation. L'α-acétolactate et l'α-acéto-hydroxy-butyrate, qui sont respectivement les intermédiaires de la voie de synthèse de la leucine, de la valine et de l'isoleucine, sont soumis à une régulation très complexe. Ils sont produits par trois enzymes indépendantes (IlvBN, IlvGM et IlvIH), l'étape ultérieure dépendante de l'enzyme IlvC est contrôlée indépendamment par un régulateur positif qui active le promoteur ilvC en présence du substrat de l'enzyme, en l'occurrence l'α-acétolactate ou l'α-acéto-hydroxybutyrate. le contrôle du pool d'α-acétolactate et d'α-acéto-hydroxy-butyrate semble donc être l'objet d'une attention particulière chez *E. coli*.

Bien qu'aucun travail exhaustif n'ait été réalisé, le contrôle génétique de ces gènes apparaît compliqué et leur organisation totalement différente de celle décrite chez d'autres bactéries comme *Bacillus subtillis* (5), *Staphylococcus aureus* (6) et *Lactococcus lactis;* chez cette dernière bactérie, tous les gènes sont assemblés en un opéron.

Des études ont montré que la croissance de *L. lactis subsp. lactis* est inhibée par un excès de leucine ou d'isoleucine en l'absence de valine. La séquence en amont de l'opéron incluant son promoteur contient les structures typiques d'un mécanisme d'atténuation répondant à la leucine; cette disposition pourrait expliquer en partie l'inhibition par la leucine.

Les Inventeurs montrent à présent que l'inhibition par la leucine n'est pas le résultat de cette régulation, mais de manière surprenante, dépend de l'activation d'une enzyme codée par un gène en aval des gènes de structures de la voie de synthèse des acides aminés branchés. Ce gène homologue à celui d'une α-acétolactate décarboxylase est probablement impliqué dans le contrôle du pool d'α-acétolactate durant la synthèse des acides aminés branchés.

La présente invention a pour objet le clonage, la caractérisation et le séquencage de la région d'ADN de *L. lactis subsp. lactis* portant le gène codant pour l'α-acétolactate décarboxylase, dénommé ci-après ald. Ce travail a été réalisé à partir de la souche NCDO2118 (AFRC, Institute of Food Research Reading Laboratory, Shinfield, Reading Berks, Royaume-Unis). La région étudiée comporte 3 gènes, assemblés sur un fragment de 1,620 kb, selon l'ordre suivant : ilvA, aldB, aldR.

Parmi ces gènes, l'invention concerne plus particulièrement d'une part une séquence d'acide nucléique codant pour un polypeptide possédant une activité α-acétolactate décarboxylase, et d'autre part une séquence d'acide nucléique codant pour un polypeptide capable d'activer une séquence d'ADN codant pour un polypeptide capable de transformer l'α-acétolactate en acétoine.

L'enzyme α-acétolactate décarboxylase présente un intérêt tout particulier puisqu'elle est responsable, chez *L. lactis subsp. lactis*, de la décarboxylation directe de l'α-acétolactate en acétoine.

Les techniques utilisées pour le clonage et le séquençage de l'acide nucléique de l'invention seront plus particulièrement décrites dans la description détaillée de l'invention.

Il sera fait référence dans ce qui suit aux tableaux et aux figures dans lesquels :
- le tableau I indique les souches bactériennes, les plasmides et phages utilisés pour le clonage des gènes ald de *L. lactis subsp*. *lactis;*
- la figure 1 représente la structure de la région d'ADN de la souche NCDO2118 de *L. lactis subsp. lactis* portant les gènes ald;
- la figure 2 représente la séquence nucléotidique ainsi que la séquence polypeptidique, déduite de cette séquence nucléotidique, de la région d'ADN de la souche NCDO2118 de *L. lactis subsp. lactis* portant les gènes ald;
- la figure 3 représente l'alignement des protéines possédant une activité α-acétolactate décarboxylase de *B. brevis* et *E. aerogenes* avec l'α-acétolactate décarboxylase de *L. lactis;*
- la figure 4 représente les structures secondaires des transcrits présents entre les gènes ilvA et aldB;
- la figure 5 représente la cartographie des transcrits couvrant la région des gènes aldBR;

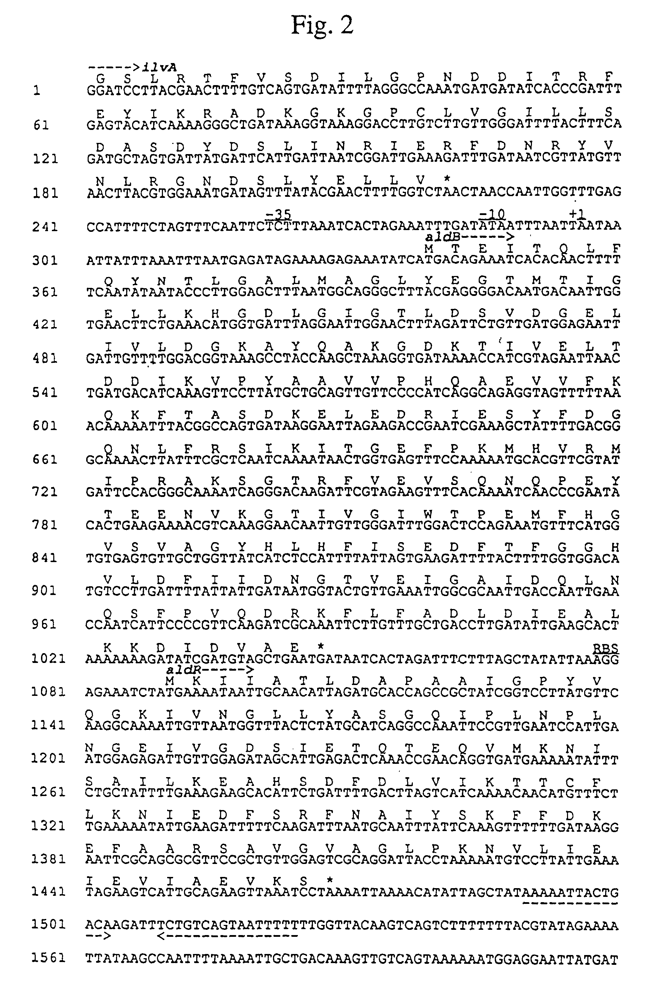

Les travaux de recherche menés sur l'ADN de la figure 2, ainsi que sur le polypeptide déduit de ce dernier, conduisent aux observations suivantes :
Trois gènes sont présents dans la région d'ADN représenté à la figure 2 :
- la fin du gène ilvA de 1539 nucléotides situé entre les nucléotides 1 et 220, et codant pour un polypeptide de 513 acides aminés;
- le gène aldB de 708 nucléotides situé entre les nucléotides 338 et 1045, et codant pour un polypeptide de 236 acides aminés possédant une activité α-acétolactate décarboxylase;
- le gène aldR de 378 nucléotides situé entre les nucléotides 1089 et 1466, et codant pour un polypeptide de 126 acides aminés; aldR constitue le gène activateur de l'expression du gène aldB.

L'invention concerne donc tout acide nucléique comprenant tout ou partie de la séquence d'ADN de la figure 2 codant, pour un polypeptide capable de transformer l'α-acétolactate en acétoine, et/ou, pour un polypeptide capable d'activer une séquence d'ADN codant pour un polypeptide capable de transformer l'α-acétolactate en acétoine.

L'invention concerne plus particulièrement, tout acide nucléique comprenant tout ou partie de la séquence d'ADN délimitée par les nucléotides situés aux positions 338 et 1045 et codant pour un polypeptide capable de transformer l'α-acétolactate en acétoine, ainsi que tout acide nucléique comprenant tout ou partie de la séquence d'ADN délimitée par les nucléotides situés aux positions 1089 et 1466 et codant pour un polypeptide capable d'activer une séquence d'ADN codant pour un polypeptide capable de transformer l'α-acétolactate en acétoine.

Un acide nucléique préféré selon l'invention comprend la séquence d'ADN délimitée par les nucléotides situés aux positions 338 et 1045 codant pour un polypeptide capable de transformer l'α-acétolactate en acétoine, ainsi que la séquence d'ADN délimitée par les nucléotides situés aux positions 1089 et 1466 codant pour un polypeptide capable d'activer une séquence d'ADN codant pour un polypeptide capable de transformer l'α-acétolactate en acétoine.

L'α-acétolactate décarboxylase de *L. lactis subsp. lactis* est une protéine constituée de 236 acides aminés codée par le fragment d'ADN délimité par les nucléotides situés aux positions 338 et 1045 de la figure 2. Son expression est activé par le produit du gène aldR contigu de aldB et délimité par les nucléotides situés aux positions 1089 et 1466.

L'invention a également pour objet tout fragment des acides nucléiques précédents codant pour un polypeptide susceptible de posséder des propriétés enzymatiques du type de celle de l'α-acétolactate décarboxylase et donc capable de transformer l'α-acétolactate en acétoine, ou encore codant pour un polypeptide susceptible d'activer la séquence d'ADN codant pour ledit polypeptide capable de transformer l'α-acétolactate en acétoine.

L'invention concerne aussi les acides nucléiques précédents dont les séquences sont modifiées dès lors que les polypeptides codés par ces acides nucléiques conservent leurs propriétés enzymatiques du type α-acétolactate décarboxylase ou acivatrice d'un gène codant pour un produit présentant des propriétés enzymatiques du type α-acétolactate décarboxylase.

De telles modifications, de manière non limitative, conduisent par exemple à des acides nucléiques variants qui se distinguent de l'acide nucléique de l'invention par addition et/ou suppression d'un ou de plusieurs nucléotides et/ou modification d'un ou plusieurs nucléotides.

Ainsi, l'invention concerne encore les acides nucléiques codant pour l'α-acétolactate décarboxylase de bactéries du genre lactococcus, telles que *L. plantarum, L. rafinolactis* et *L. lactis* comme *L. lactis subsp. cremoris;* lesquelles séquences d'ADN présentent une forte homologie avec la séquence nucléique du gène aldB.

Avantageusement, ces acides nucléiques sont identifiés, le cas échéant sélectionnés, par hybridation génétique avec une ou plusieurs sondes nucléiques issues de la séquence d'aldB, dans des conditions de stringence moyennes.

A titre d'exemple, un procédé d'identification d'un tel acide nucléique comprend les étapes suivantes :
- extraction de l'ADN de la bactérie;
- digestion de cette ADN par une ou plusieurs enzymes de restriction, telle que EcoR1;
- transfert des fragments d'ADN obtenus sur membrane de nitrocellulose;
- hybridation avec la séquence complète d'aldB, dans une solution d'hybridation dont la composition est : 6 X SSC, environ 10 % formamide, 5 X Denhardt, 100 ml de tampon phosphate pH7.

L'invention concerne également les polypeptides comprenant tout ou partie de la séquence polypeptidique de la figure 2 codée par la séquence d'ADN délimitée par les nucléotides situés aux positions 338 et 1045, lesquels polypeptidique possèdent une activité α-acétolactate décarboxylase, ainsi que tous les polypeptides susceptibles de posséder une activité α-acétolactate décarboxylase et qui sont codés par les fragments d'ADN susmentionnés.

L'invention a aussi pour objet toute séquence polypeptidique issue de la séquence précédente, dès lors que ce polypeptide possède des propriétés enzymatiques du type de celle de l'α-acétolactate décarboxylase.

L'invention se rapporte également aux polypeptides comprenant tout ou partie de la séquence polypeptidique de la figure 2 codée par la séquence d'ADN délimitée par les nucléotides situés aux positions 1089 et 1466, lesquels polypeptidique possèdent des propriétés activatrices d'une séquence d'ADN codant pour un polypeptidique possèdant une activité α-acétolactate décarboxylase.

Les polypeptides précédents peuvent être modifiés dès lors qu'il conservent les propriétés enzymatiques et activatrices définies précédemment. Par exemple, de manière non limitative, des polypeptides entrant dans le cadre de l'invention peuvent se distinguer des ceux définis ci-dessus par addition et/ou suppression d'un ou de plusieurs acides aminés et/ou modification d'un ou plusieurs acides aminés.

L'homme du métier dispose des moyens lui permettant d'identifier ceux des polypeptides de séquences plus courtes qui entrent dans le champ de l'invention. Un moyen général lui permettant de procéder à cette identification, consiste à traiter les polypeptide délimitée par les nucléotides situés aux positions 338 et 1045 ou par les nucléotides situés aux positions 1089 et 1466 de la figure 2, avec une protéase clivant le polypeptide au niveau d'un site précis, puis de séparer les fragments obtenus et de les tester pour leur activité enzymatique vis-à-vis de l'α-acétolactate, ou leur activité activatrice vis-à-vis du gèn' codant pour l'α-acétolactate décarboxylase.

Un autre moyen permettant d'identifier des régions du polypeptide essentielle à l'activité α-acétolactate décarboxylase ou à l'activation du gène codant pour l'α-acétolactate décarboxylase, consiste à cliver l'acide nucléique correspondant à aldB ou à aldR, par exemple à l'aide d'une ou plusieurs enzymes de restriction, avant son introduction dans l'hôte cellulaire ou le vecteur d'expression utilisé pour la production d'une protéine présentant une activité α-acétolactate décarboxylase. L'acide nucléique tronqué peut ainsi être testé, pour sa capacité à exprimer effectivement un produit possédant une activité α-acétolactate décarboxylase ou des propriétés activatrices du gène aldB, ou au contraire ne les possédant plus.

Les polypeptides précédents présentant une activité α-acétolactate décarboxylase sont utiles pour dégrader l'α-acétolactate en acétoine.

L'invention a donc également pour objet tout acide nucléique recombinant contenant un acide nucléique du type sus-indiqué codant pour l'α-acétolactate décarboxylase ou pour un polypeptide activateur d'une séquence d'ADN codant pour l''α-acétolactate décarboxylase, ou tout autre polypeptide susceptible de posséder ces propriétés, associé avec un promoteur et/ou un terminateur de transcription reconnu par les polymérases de la cellule hôte dans laquelle ledit acide nucléique recombinant est introduit.

L'introduction de l'acide nucléique recombinant dans une cellule hôte peut être réalisée à l'aide de vecteurs du type plasmide aptes a se répliquer dans la cellule hôte et à y permettre l'expression de la séquence codant pour l'enzyme, ou encore l'acide nucléique recombinant peut être introduit directement dans le génome par des techniques du génie génétique connues de l'homme du métier.

Les hôtes dans lesquels l'acide nucléique recombinant est susceptible d'être introduit sont préférentiellement des microorganismes. Plus particulièrement, il s'agit de tous microorganismes chez lesquels l'enzyme α-acétolactate docarboxylase est absente, comme les levures. Cette transformation du microorganisme permet de dégrader l'α-acétolactate en acétoine neutre au goût, et est donc particulièrement indiqué dans le cas de productions où le diacétyle n'est pas souhaité comme pour la fabrication de la bière.

L'invention concerne également les applications de ces acides nucléiques pour la conception de nouveaux modes d'inactivation du gène codant pour l'α-acétolactate décarboxylase chez les bactéries de type *L. lactis,* en vue de favoriser chez ces dernières la production de diacétyle.

Ainsi, dans les cas ou la production de diacétyle est recherchée, en raison du caractère aromatique de ce composé, l'inactivation du gène aldB permet d'augmenter la production de diacétyle. En effet, l'α-acétolactate décarboxylase détourne la majeure partie du précurseur du diacétyle, l'α-acétolactate, vers l'acétoine qui est un composé neutre au goût. L'invention concerne en conséquence la préparation de bactéries du type *L. lactis,* d'origine végétale ou laitière, dont le gène aldB est inactivé, ces bactéries étant alors capables de surproduire du diacétyle. L'inactivation du gène aldB peut être réaliser soit en agissant directement sur le gène aldB, soit indirectement, en agissant sur son gène activateur aldR.

Un exemple préféré de procédé de préparation de bactérie du type *L. lactis* dont le gène aldB et/ou le gène aldR sont inactivés, lesquelles bactéries étant donc capables de surproduire du diacétyle, consiste :
- à choisir deux segments d'au moins 300 pb situés soit, de part et d'autre ou dans l'acide nucléique délimité par les nucléotides situés aux positions 338 et 1045, soit de part et d'autre ou dans l'acide nucléique délimité par les nucléotides situés aux positions 1089 et 1466,
- à préparer un acide nucléique dont chaque extrémité est constituée de l'un des segments précédents,
- à introduire ledit acide nucléique dans un vecteur, lequel est à son tour introduit dans la souche hôte afin d'obtenir par une technique d'échange d'allèles ou de double recombinaison des batéries dont le gène aldB ou le gène aldR, sauvages, sont inactivés.

L'inactivation du gène aldB ou du gène aldR selon le procédé précédent peut être réalisée par délétion de l'un ou l'autre de ces gènes au moyen d'un acide nucléique essentiellement constitué desdits segments d'au moins 300 pb, ou encore par remplacement de l'un ou l'autre de ces gènes par un gène muté, au moyen d'un acide nucléique délimité par les segments d'au moins 300 pb entre lesquels est disposé un gène muté.

On préfère tout particulièrement utiliser pour réaliser l'échange d'allèles ou la double recombinaison des segments de 1 kb.

En outre, afin de sélectionner les bactéries dont le gène aldB est inactivé, on peut introduire au sein de l'acide nucléique et entre les segments un marqueur tel qu'un gène étranger facilement décelable ou encore un séquence nucléique pouvant être révélée par hybridation.

De manière avantageuse, cette inactivation du gène aldB chez les bactéries *L. lactis,* est combinée à une activation chez ces bactéries du flux métabolique menant à l'α-acétolactate.

Une telle activation peut être réalisée par tout moyen chimique ou biologique permettant de favoriser chez ces bactéries la transformation du pyruvate en α-acétolactate; un exemple de moyen biologique consiste à transformer ces bactéries en introduisant directement dans le génome ou via un vecteur type plasmide, un gène codant pour une α-acétolactate synthase.

L'invention concerne enfin un procédé de sélection de bactéries de type *L. lactis*, d'origine végétale ou laitière, présentant une déficience α-acétolactate décarboxylase et donc surproduisant du diacétyle.

Lequel procédé comprend les étapes suivantes:
- le traitement d'une bactérie, éventuellement préalablement sélectionnée sur un milieu de culture, de façon à rendre les acides nucliques qu'elle contient apte à s'hybrider avec une sonde,
- la mise en contact lesdits acides nucléiques avec une sonde ou mélange de sonde, éventuellement marquée, constituée de tout ou partie d'un acide nucléique selon l'invention, dans des conditions permettant la formation de complexe d'hybridation entre la ou les sondes et les acides nucléiques provenant de la bactérie,
- la mise en évidence par tous moyens appropriés les hybrides éventuellement formés afin de déterminer la présence de mutation s'opposant à la formation desdits hybrides et indiquant une déficience α-acétolactate décarboxylase.

D'autres caractéristiques de l'invention apparaîtront dans la description qui suit se rapportant d'une part au clonage, à la caractérisation et au séquençage de la région de l'ADN de *L. lactis subsp. lactis* portant les gènes codant pour l'α-acétolactate décarboxylase, et d'autre part à l'introduction dans un hôte cellulaire d'un acide nucléique selon l'invention codant pour un polypeptide présentant une activité α-acétolactate décarboxylase, ou à la modification des gènes aldB et/ou aldR de bactéries *L. lactis* afin de favoriser chez ces dernières la production de diacétyle.

### I - CLONAGE SEQUENCAGE ET ORGANISATIONS DES GENES ALD.

### A) MATÉRIEL ET MÉTHODES.

### 1) Souches bactériennes, plasmides et milieux de culture.

Les souches bactériennes, plasmides et phages utilisés pour le clonage des gènes sont rapportés dans le tableau I ci-après.

**Tableau I**

| Souches et plasmides | Caractéristiques | Références bibliographiques ou sources |
|---|---|---|
| *L. Lactis subsp. lactis* | | |
| NCDO2118 | isolat naturel | NCDO |
| IL4460 | aldB::tetR | |
| IL4471 | aldR::tetR | |
| IL4173 | NCDO2118 délété de tout l'opéron de la voie de synthèse des acides aminés branchés | |
| | | |
| *E. coli* | | |
| | | |
| TG1 | supE thi D(lac-proAB) hsdD5 F⁺ traD36 proAB lacI zΔ15 | (Gibson)^{b} |

| Plamides | | |
|---|---|---|
| pIL253 | Em^{R}, 4.9-kb | |
| pIL500 | Fragment XbaI de 18.5-kb du du chromosome de L. lactis dans pIL253 | |
| pIL593 | Fragment BamH1 XbaI de 3,8-kb de pIL500 dans pIL253 | |
| pIL557 | Fragment BamH1 XbaI de 3,8-kb de pIL500 dans pBluescript | |
| pIL540 | Fragment BamH1 EcoR1 de 1,2-kb de pIL500 dans pBluescript | |
| pIL4460 | pIL557 avec tetM dans PstI | |
| pIL4471 | pIL557 avec tetM dans NsiI | |
| pBluescript | Ap^{R}, M13 ori, pBR322 ori | (Stratagene) |

Dans le tableau I, (^{b}) indique la référence bibliographique : T. J. Gibson, Ph. D thesis, University of Cambridge, Cambridge, England.

*L. lactis subsp. lactis* NCDO2118 a été mise en culture à 30°C dans un milieu M17 (8) dans lequel le lactose est remplacé par du glucose ainsi que dans un milieu défini chimiquement (CMD) (9). *Escherichia coli* a été cultivée sur un milieu Luria-Bertani (LB) ou dans le milieu minimum M63 (10) à 37°C. *Bacillus subtillis* a été cultivé à 37°C sur un milieu LB ou dans un milieu minimum MM (11). Si nécessaire, de l'erythromycine (5 µg/ml pour *L. lactis*) de l'ampicilline (50 g/ml pour *E. coli*) ou de la tétracycline (10 g/ml pour *L. lactis* et *E. coli*) sont ajoutés au milieu.

### 2) Clonage moléculaire et manipulation d'ADN

Les plasmides et l'ADN chromosomique ont été préparés selon les techniques connues de l'Art antérieur (12a, 12b). La transformation des cellules de *L. lactis* a été réalisée selon une procédure d'électroporation standard (13). Les Southern blots et les hybridations d'ADN ont été réalisés comme décrit par Maniatis et al. (10). Les sondes nucléique ont été préparées en utilisant du (α³²P)-dCTP avec un kit de nick translation conformément aux recommandation du fournisseur. Les autres techniques moléculaires ont été mises en oeuvre comme décrit par Maniatis et al. (10).

### 3) Analyse de la séquence d'ADN

Les clones d'*E. coli* mis en oeuvre pour le séquençage ont été obtenus par sous-clonage de fragments d'ADN dans les plasmides de la famille pBluescript et en utilisant l'exonucléase III et la nucléase "mung bean" de la Société Stratagene, afin de générer une série de clones contenant des fragments d'ADN se recouvrant.

L'ADN a été ensuite séquencé en utilisant le kit "Taq DyeDeoxy Terminator Cycle Sequencing" et le "Sequencer 370A" commercialisés par la Société Applied Biosystem. Les séquences nucléiques ont été déterminées deux fois sur les deux brins. Les séquences nucléotidiques et polypeptidiques ont été analysées avec les logiciels "BISANCE" et "GCG" de l'Université du Wisconcin. Les séquences protéiques ont été alignées avec un logiciel "MULTALIN" (14).

### 4) Hybridation en northern blot

L'ARN a été préparé à partir de cellules cultivées de manière exponentielle sur un milieu CMD supplémenté en acides aminés branchés, lavé trois fois puis transféré sur un milieu CMD avec ou sans acides aminés branchés pendant 20 minutes. L'ARN total a été préparé comme décrit précédemment (15). Le marqueur d'ARN (BRL) a été utilisé pour estimer la taille des ARNm. L'hybridation en northern blot a été réalisée comme décrit par Maniatis et al. (10).

### 5) Remplacement de gène par double crossing over

Le gène tetM de Tn1545 a été cloné au site Pst1 du gène aldB ou au site NsiI du gène aldR dans le plasmide pIL557 contenant la région ald de pIL593. Cette construction a été introduite dans le plasmide Ts55. Le remplacement du gène chromosomique par le gène tronqué par double crossing over a été effectué comme décrit précédemment pour *Bacillus subtilis* (16). Les souches mineures mutées dans les gènes aldB et aldR ont été dénommées respectivement IL4460 et IL4471.

### B) RÉSULTATS

### 1) Inhibition par la leucine

La souche NCDO2118 de *L. Lactis subsp. lactis* est capable de croître dans un milieu CMD sans acides aminés branchés. Lorsque la leucine est ajoutée à ce milieu, les cellules ne peuvent plus pousser, la croissance est retrouvée dans un milieu CMD supplémenté avec de la leucine et de la valine.

En conséquence, il apparaît que la leucine inhibe la synthèse de la valine mais pas celle de l'isoleucine.

### 2) Identification des gènes responsables de l'inhibition par la leucine

Les gènes impliqués dans la voie de synthèse de acides aminés branchés ont été clonés dans un fragment de 18,5 kb. Ce fragment contient l'ensemble des gènes de structure impliqués dans la voie de synthèse de acides aminés branchés à l'exception du gène codant pour une transaminase, correspondant à la dernière étape de la voie de synthèse.

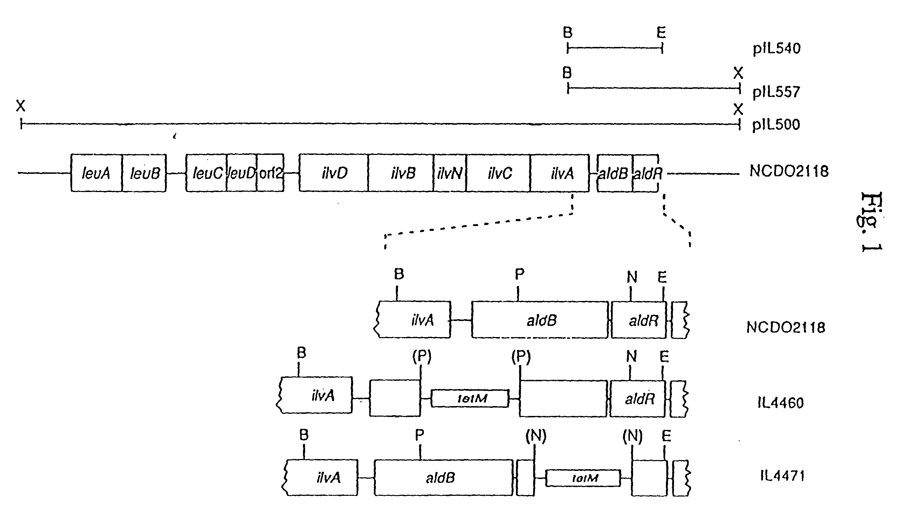

La figure 1 représente l'organisation de l'ensemble des gènes de la voie de synthèse des acides aminés branchés y compris les gènes ald, et les constructions utilisées :
- la partie supérieure de la figure 1 indique les inserts des différents plasmides utilisés,
- la partie médiane de la figure 1 représente l'organisation de l'ensemble des gènes de la voie de synthèse des acides aminés branchés y compris les gènes ald,
- la partie inférieure de la figure 1 indique les remplacements de gènes sur le chromosome.

Le nom des souches et des plasmides sont indiqués à gauche; les enzymes de restrictions sont représentés par les lettres suivantes : X pour XbaI, B pour BamHI, E pour EcoRI, P pour PstI et N pour NstI.

Aucune structure typique rho-indépendante de fin de transcription n'a été trouvée après le gène ilvA. En outre la séquence N terminale du prochain ORF a été trouvé homologue au gène α-ALDC de *Bacillus brevis* (17) et de *Enterobacter aerogenes* (18). Cette enzyme transforme l'α-acétolactate, le premier intermédiaire de la synthèse de la leucine et de la valine, en acétoine. En conséquence, l'expression de ce gène interfère avec la synthèse de la valine.

Afin de comparer cette hypothèse avec celles impliquant une régulation par atténuation ou une inhibition par feed-back, deux mutants spontanément résistant à la leucine ont été isolés. Ces deux mutants ont été transformés avec les plasmides pIL500 ou pIL593; pIL500 contenant l'opéron complet de la chaîne de synthèse des acides aminés branchés et un fragment de 3,5 kb en aval du gène ilvA, et pIL593 contenant un fragment de 3,8 kb commençant à la fin du gène ilvA.

On observe que ces deux plasmides restaurent chez ces souches mutantes un phénotype leucine sensible, indiquant que ce phénotype est associé au fragment d'ADN contenant le gène homologue à α-ALDC.

Pour vérifier que le plasmide pIL583 contient un gène codant pour une α-ALDC, ce plasmide a été introduit dans une souche naturellement dépourvue d'activité α-ALDC. En absence du plasmide, cette souche produit une grande quantité de diacétyle (0,51 mM) en raison de l'accumulation de l'α-acétolactate, et une quantité négligeable d'acétoine. Par contre les dérivés de cette souche contenant le plasmide pIL583 ne produisent que très peu de diacétyle (0,1 mM) au profit de l'acétoine (5 mM). Ceci montre que le plasmide permet l'expression d'une enzyme transformant l'α-acétolactate en acétoine.

### 3) Analyse de séquences

La séquence de 1,5 kb en amont du gène ilvA est représentée à la figure 2, cette séquence nucléique et la séquence en acides aminés qui en est déduite correspondent à la région ald de la souche *L. lactis* NCDO2118. Les nombres à gauche indiquent les nucléotides. les noms des gènes sont indiqués au début de la séquence d'acides aminés correspondante; les codons d'arrêts de la transcription ainsi que les sites de fixation des ribosomes sont indiqués respectivement par (*) et RBS; les séquences consensus "-10" et "-35" des promoteurs sont soulignés, la flèche (----->) indique le terminateur de transcription.

Deux ORF finissant avec un terminateur de transcription rho-indépendant ont été mis en évidence. Le premier code pour une protéine de 236 acides aminés homologue à l'acétolactate décarboxylase de *B. brevis* et de *E. aerogène* avec respectivement 34,2 % et 39,9 % d'identité et dénommé AldB. Le second, dénommé AldR, code pour une protéine de 126 acides aminés. Des recherches d'homologie avec des protéines présentes dans des bases de données n'ont révélées aucune protéine présentant des similarités significatives avec AldR.

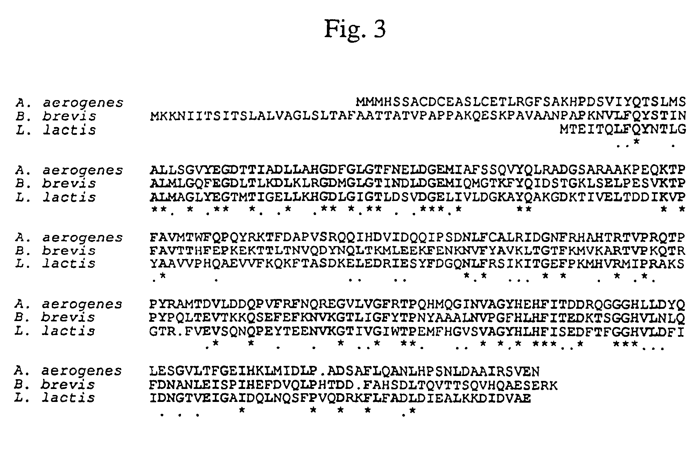

Ces résultats sont issus de l'étude d'homologies, dont les résultats d'alignement des protéines α-ALDC de *B. brevis* et *E. aerogenes* avec La protéine AldB de *L. lactis* sont représentés à la figure 3. Pour cette étude le logiciel de Mutlalin (14) a été utilisé. Dans la figure 3 les symboles (*) indiquent les acides aminés communs à toutes les protéines, les symboles (.) indiquent les substitutions conservées; les acides aminés représentés en caractères gras sont présents dans au moins deux protéines; les nombres entre parenthèses à droite correspondent à la taille des protéines.

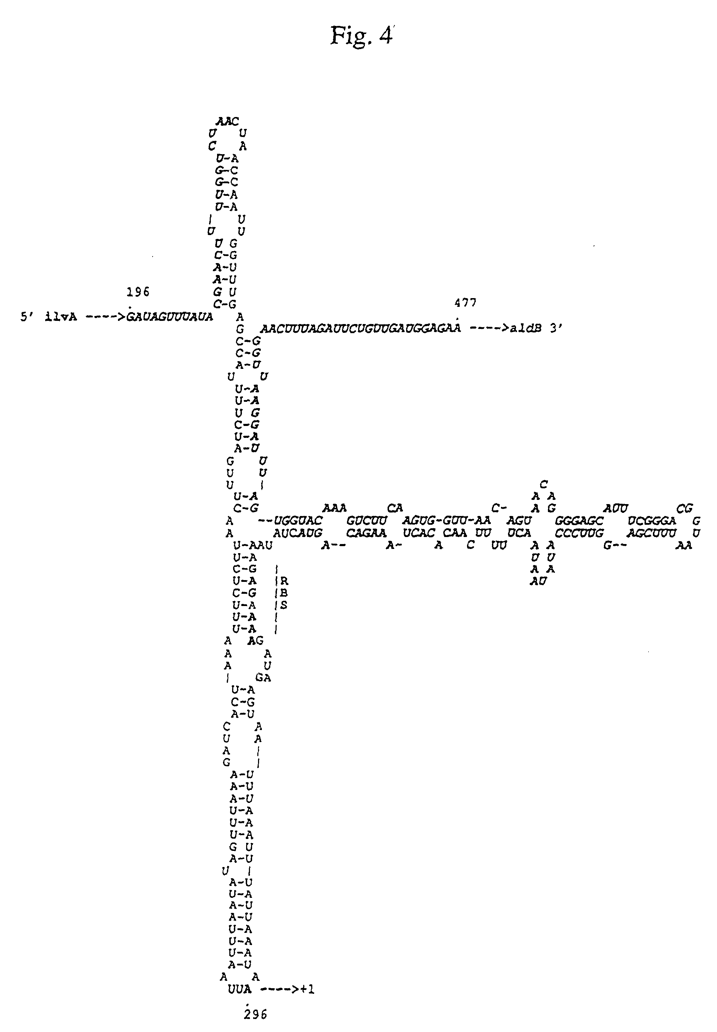

La figure 4 indique les structures secondaires potentielles présentent entre les gènes ilvA et ilvB des transcrits commençant en p1 et p2. Les caractères gras indiquent les sites de fixation des ribosomes du gène aldB; la flèche indique le début de transcription de l'opéron aldBR depuis p3; les séquences codant pour IlvA et AldB sont représentées en caractères italiques gras.

Les études informatiques montrent que l'ARN entre les gènes ilvA et aldB peut être replié selon une structure secondaire forte. Cette structure cache le site de fixation des ribosomes et le codon de départ de aldB. Aucune séquence consensus correspondant au promoteur végétatif de *L. lactis* n'a été mise en évidence dans cette région.

### 4) Analyse transcriptionelle des gènes ald

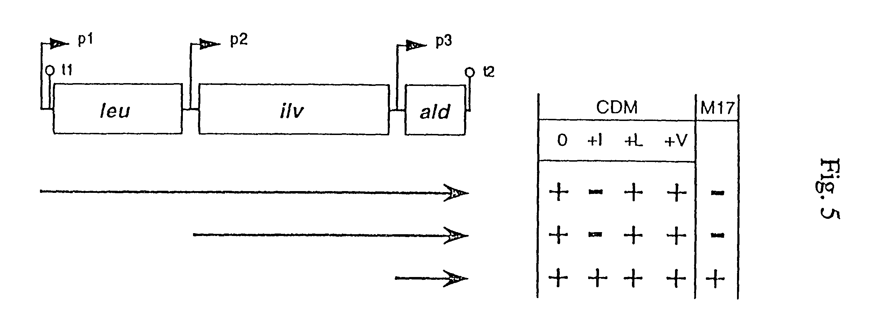

La figure 5 représente les transcrits couvrant l'ensemble aldBR; p1, p2 et p3 indiquent les promoteurs de transcription, et t1 et t2 indiquent les terminateurs de transcription; les flèches représentent les différents ARN détectés en northern blot. Le tableau à droite de la figure 5 indique la présence (+) ou l'absence (-) des transcrits correspondants dans les cellules cultivées sur différents milieux.

Des Northern blot avec l'ARN extrait de cellules cultivées sur un milieu CMD sans acides aminés branchés montrent que les gènes ald pourraient être transcrits depuis trois promoteurs p1, p2 et p3 et terminés à la même extrémité 3' en t2. Les tailles de ces transcrit sont respectivement de 14500, 800 et 1200 nucléotides.
Le transcrit initié en p3 est présent que les cellules aient été cultivées sur un milieu CMD avec ou sans les acides aminés branchés ou sur un milieu M17, alors les transcrits commençant depuis p1 et p2 sont présents uniquement dans les souches cultivées sur un milieu CMD sans acide aminé branché. La transcription depuis p1 et p2 diminue fortement en présence d'isoleucine dans le milieu CMD alors que la leucine et la valine n'ont pas d'effet détectable sur la transcription.

Le début de transcription depuis p3 suivi par extension d'amorces en position 296 sur la figure 2 correspond à la boucle d'une structure secondaire potentiel de l'ARNm entre les gènes ilvA et aldB. La séquence consensus "-10" est semblable à celle de la séquence promotrice végétative de *L. lactis,* alors que la séquence consensus "-35" ne l'est pas.

### 5) Rôle des gènes aldBR dans la voie de synthèse de la leucine et de la valine

Les deux gènes de l'ensemble ald (aldB et aldR) ont été interrompus avec un gène tetM de Tn1545 pour préparer les mutants IL4460 etIL4471 indiqués à la figure 1.

La souche mineure aldB, IL4460, n'est alors plus sensible à la leucine, mais la souche mineure aldR, IL4471, reste sensible à la leucine. Le phénotype leucine sensible a été restauré chez le mutant IL4460 lorsque le gène aldB était présent en trans sur le plasmide pIL593.

### C) DISCUSSION

L'opéron contenant les gènes de structure de la voie de biosynthèse des acides aminés branchés, inclue deux gènes supplémentaires à son extrémité 3'. L'un des ces gènes ald code pour une α-acéolactate décarboxylase responsable de la décarboxylation de l'α-acétolactate en acétoine. Cette enzyme est significativement homologue à celle de *E. aerogenes* et *B. brevis.* Comme l'enzyme de *E. Aerogenes,* elle ne contient pas de peptide leader nécessaire à l'exportation de la protéine, comme cela est le cas pour *B.brevis* et *B. subtilis*, suggérant qu'il s'agit d'une enzyme intracellulaire (17).

Le gène aldB qui code pour une enzyme dégradant le premier intermédiaire de la voie de synthèse de la leucine et de la valine est lié à l'opéron de la chaîne de synthèse des d'acides aminés branchés de manière surprenante, puisque son activité peut interférer avec ladite biosynthèse. Comme cela a été montré par des techniques de remplacement du gène aldB, l'inhibition par la leucine de la synthèse de la valine chez *L. lactis subsp. lactis* NCDO2118 est le résultat de l'activité de la protéine AldB. Par ailleurs, aucune inhibition de la synthèse de leucine n'est observée en présence de valine, posant la question de la voie de régulation.

L'analyse des transcrits révèle que le gène ald pourrait être transcrit à partir de trois promoteurs p1, p2 et p3 localisés respectivement en aval des gènes leu, ilv et ald. Alors que p1 et p2 sont réprimés par l'isoleucine, p3 n'est pas sous le contrôle de la voie de synthèse des acides aminés branchés. Une telle régulation ne peut expliquer l'inhibition par la leucine, et de plus l'inhibition par la leucine ne peut dépendre du transcrit p3, comme l'inactivation de p3 par remplacement de aldR par tetM ne lève pas la sensibilité à la leucine.

Lorsque le gène aldB est transcrit depuis p1 et p2, l'ARNm peut se replier en une structure secondaire forte empêchant la traduction du gène aldB. Il est possible qu'en présence de leucine, cette structure soit déstabilisée par un mécanisme non encore compris.

Par ailleurs, le transcrit p3 n'inclue pas cette structure ce qui suggère l'absence de ce contrôle post-transcriptionnel de l'enzyme AldB quand ce promoteur est induit. La production d'α-acétolactate décarboxylase depuis p3, qui est sous le contrôle de AldR peut donc ne pas être liée aux variations du pool d'α-acétolactate durant le métabolisme de la voie de synthèse des acides aminés branchés. Deux types de métabolisme peuvent conduire à la production d'une important quantité d'acétoides. Le premier est rencontré chez *Bacillus* et *Klebsiella* qui possèdent deux α-acétolactates synthases, l'une impliquée dans la voie de synthèse des acides aminés branchés et l'autre dans le catabolisme du pyruvate en acétoine (19, 20).Chez *Bacillus* l'α-acétolactate synthase dAldB sont organisés en un seul opéron qui est transcrit dans une phase stationnaire (21).

Les bactéries acide lactique comme *L. Lactis subsp. lactis biovar diacetylactis, Leuconostoc lactis, Lactobacillus lactis subsp. lactis* et *Lactobacillus plantarum* sont aussi capables de produire une quantité importante d'acétoine. Ce métabolisme est dépendant d'une source exogène de citrate qui est prise en charge par un plasmide codant pour une perméase. Comme la plupart des souches *L. Lactis* ne métabolisent pas le citrate, le rôle exact de l'enzyme AldB, lorsqu'elle est transcrite de p3 n'est pas clair.

Outre son rôle dans la régulation du pool d'α-acétolactate durant la synthèse des acides aminés branchés, l'enzyme α-acétolacte décarboxylase pourrait être impliquée dans des voies de synthèse de métabolites secondaires. Cette enzyme est soigneusement régulée par un activateur, comme IlvC chez *E. Coli*, une telle régulation de l'enzyme impliquée dans la transformation de l'α-acétolacte pourrait être importante pour éviter son oxydation spontanée en diacétyl qui est potentiellement toxique.

### II - INTRODUCTION D'UN ACIDE NUCLEIOUE CODANT POUR L'α-ACÉTOLACTATE DECARBOXYLASE DE L. LACTIS SUBSP, LACTIS DANS LA LEVURE

### 1) Fusion du gène aldB et du promoteur ADH1 de la levure

Le promoteur ADH1 de Saccharomyces cerevisiae est amplifié selon un processus classique d'amplification génétique dit par PCR à l'aidé des deux oligonucléotides amorces suivants : - CGATTTTTTTCTAAACCGTGG, - CCATATGTATATGAGATAGTTGATT, puis traité à la polymérase T4 pour créer des bouts francs à ses extrémités, et cloné dans pBluescript au site EcoRV dans *E. coli.* Un site NdeI est créé en aval du promoteur ADH1. La séquence codante pour la protéine AldB de *L. lactis subsp*. *lactis* est ensuite amplifiée selon un processus d'amplification génétique dit par PCR à partir de deux oligonucléotides amorces; l'un débutant au codon de démarrage de traduction du gène aldB et dont la séquence est : ATGACAGAAATCACACAACT, et l'autre en sens inverse à la fin du gène aldB, et dont la séquence est :

CTAAAGAAATCTAGTGATTA.

Après traitement à la polymérase T4, le produit amplifié est ensuite cloné en aval du promoteur ADH1 de la construction précédente dans *E. coli* au site NdeI traité à la nucléase S1. Après avoir choisi la construction dans laquelle le fragment inséré se trouve dans l'orientation correcte pour être exprimé par le promoteur ADH1, cette construction est clonée à l'aide des enzymes EcoRI et HindIII dans un vecteur approprié à la levure. Ce vecteur peut être réplicatif comme YEP24 (commercialisé par la Société Biolabs) ou intégratif comme YIP5 (commercialisé par la Société Biolabs).

### 2) Introduction du gène aldB sous contrôle du promoteur ADH1 dans la levure

a) Le vecteur YEP24 est restreint par SmaI, déphosphorylé à l'aide de la phosphatase alcaline de veau. Pour y insérer le gène aldB sous contrôle du promoteur ADH1, le plasmide traité est ligué en présence du fragment EcoR1-HindIII de la construction précédente, et traité à la polymérase T4. Le mélange de ligation est ensuite transformé dans *E. coli* et les clones contenant la construction désirée sont sélectionnés. La construction peut alors être testée après transformation de la levure.
b) Le vecteur YIP5 est restreint par l'enzyme ApaI, traité à la polymérase T4 et déphosphorylé à la phosphatase alcaline de veau. Il est ensuite ligué avec le fragment EcoR1-HindIII de la construction précédente et traité à la polymérase T4. Le mélange de ligation est ensuite transformé dans *E. Coli* et les clones contenant le gène aldB sous contrôle du promoteur ADH1 inséré dans le gène URA3 de *S. cerevisiae* sont sélectionnés. Cette construction est introduite par transformation sur le chromosome de la levure par double recombinaison au site URA3.

3) Des clones exprimant un niveau élevé d'α-acétolactate décarboxylase sont ainsi préparés et peuvent être utilisés pour réduire la quantité de diacétyle produit par la levure.

### III - DESTRUCTION DE L'ACTIVITE α-ACETOLACTATE DECARBOXYLASE CHEZ L. LACTIS

Chez *L. lactis,* lorsque la protéine codée par le gène aldB est exprimée, celle-ci transforme l'α-acétolactate en acétoine. Cette transformation rapide se fait au détriment de la transformation lente de l'α-acétolactate en diacétyle. En conséquence, la réduction de l'activité de la protéine AldB peut être avantageuse lorsque l'on cherche à favoriser la production de diacétyle; ainsi certains mutants naturels dépourvus de l'activité α-acétolactate décarboxylase surproduisent du diacétyle. La délétion de tout ou partie du gène aldB ou le remplacement de la séquence naturelle par une séquence modifiée permettent d'obtenir des mutants surproducteurs de diacétyle. La délétion de tout ou partie du gène aldB ou son remplacement par une séquence modifiée chez L. lactis, ne sont possibles que grâce aux travaux sur la séquence des gènes aldB et aldR, ayant conduit à l'acide nucléique de l'invention.

### Exemple 1 : Destruction de l'activité α-acétolactate décarboxylase de L. lactis par troncature de la séquence sauvage aldB et introduction de la séquence tronquée dans le chromosome

Un fragment d'ADN codant pour la résistance à la tétracycline est introduit au site Pst1 en position 564 de la séquence de la figure 2 dans le plasmide pIL1230. La séquence tronquée est ensuite introduite à la place du gène sauvage dans les souches NCDO2118, IL1403 et BU2-60 , ces deux dernière souches étant d'origine laitière, par une méthode classique de remplacement de gène par double recombinaison.

La construction est d'abord clonée sur le plasmide pVE6004 dont la réplication peut être bloquée par la température, puis introduite sur ce vecteur dans la souche hôte. Après passage à 37°C, température non permissive pour la réplication du plasmide, les clones contenant la construction intégrée sur le chromosome par recombinaison simple ou double sont sélectionnés par leur résistance à la tétracycline.

Dans un second temps, la population contenant la construction intégrée par deux recombinaison est enrichie en permettant l'excision des constructions non désirées à 30°C et en effectuant une nouvelle sélection pour la présence du marqueur tétracycline à 37°C. Les clones contenant la séquence nucléotidique modifiée sont vérifiés afin de confirmer qu'elles ne produisent plus d'α-acétolactate décarboxylase active.

L'introduction de cette construction dans des souches productrices de diacétyle permet d'augmenter d'environ dix fois la quantité de diacétyle produite.

Ainsi, il a été construit la souche JIM4460, dérivée par cette méthode de la souche NCDO2118, faible productrice de diacétyle. En milieu M17 sans glycérophosphate et additionné de glucose (2 %) et de citrate (0,2 %) et sous aération, la souche JIM4460 produit 0,51 mM de diacétyle contre 0,4 mM pour la souche NCDO2118. Cette production se fait à partir du pyruvate produit par la glycolyse, puisque ces souches n'utilisent pas le citrate. L'augmentation de la production de diacétyle est due à la destruction du gène AldB. En effet, la souche JIM4460 produit une quantité négligeable d'acétoine (0,06 mM) contrairement à la souche NCDO2118 (1,6 mM).

### Exemple 2 : Diminution de l'activité α-acétolactate décarboxylase de L. lactis par destruction du gène sauvage aldR codant pour un activateur du gène aldB.

Un fragment d'ADN codant pour la résistance à la tétracycline est introduit au site Pst1 en position 1166 de la séquence de la figure 2 dans le plasmide pIL1230. Cette séquence nucléotidique est ensuite introduite à la place du gène sauvage dans les souches NCDO2118, IL1403 et BU2-60 selon la méthode de double recombinaison décrite à l'exemple 1.

Les souches obtenues ne transcrivent plus de manière significative le gène codant pour l'α-acétolactate décarboxylase; ainsi l'introduction de cette construction à la place du gène sauvage correspondant dans une souche productrice de diacétyle permet de réduire considérablement l'expression de l'α-acétolactate décarboxylase et donc de favoriser la transformation de l'α-acétolactate en diacétyle au détriment de l'acétoine.

### Exemple 3 : Introduction d'une mutation dans le gène codant pour l'α-acétolactate décarboxylase de L. lactis, et conduisant à l'expression d'une protéine tronquée ou inactive.

La séquence nucléotidique de la figure 2 est modifiée par coupure du plasmide pIL540 par l'enzyme pSTI, puis action de la T4 polymérase et ligation sur lui-même. Le produit obtenu est une protéine modifiée à partir de la position 565 et tronquée de 313 acides aminés.

Cette construction est introduite dans les souches NCDO2118, IL1403 et BU2-60 par la méthode de double recombinaison décrites dans les exemples précédents.

Le plasmide pIL584 est introduit dans la souche hôte par transformation afin de rendre cette souche prototrophe pour la leucine, la valine et l'isoleucine.

Les souches dans lesquelles la séquence modifiée est présente à la place de la séquence sauvage sont sélectionnées pour leur résistance à la leucine conférée par la mutation introduite. Cette sélection est réalisée en étalant les souches à 37°C sur un milieu dépourvu d'isoleucine et de valine (Poolman, B et W. N. Konings, 1988, J. Bacteriol. 170 : 700-707). Après la perte spontanée du plasmide pIL584, la mutation introduite est vérifiée par l'absence de signal d'hybridation avec un oligonucléotide correspondant à la séquence sauvage, tel que CCTTATGCTGGAGTTGT.

La souche obtenue est dépourvue d'activité α-acétolactate décarboxylase. Ainsi, après introduction d'un plasmide portant un acide nucléique codant pour une perméase au citrate, ou pour une α-acétolactate synthase, cette souche surproduit du diacétyle.

Un certain nombre de mutants résistants à la leucine ont été obtenu à partir de la souche NCDO2118 et leur production de diacétyle a été testé dans les mêmes conditions que pour l'exemple 1. Ainsi, les souches L1, L2, L5, L6, L7 et L8 produisent de 0,43 à 0,61 mM de diacétyle et des quantités d'acétoine réduites, alors que dans les mêmes conditions, la souche NCDO2118 produit une quantité négligeable de diacétyle au profit de l'acétoine.

### Références

1 - Calvo, J. M. (1983), pp. 267-284. In Herrmman, K. M., Somerville, R. L. (Eds.); Amino acids, biosynthesis and genetic regulation, Addison-Wesley, Reading, Mass.
2 - Kohlhaw, G. B. (1983), pp. 285-299. In Herrmman, K. M., Somerville, R. L. (Eds.); Amino acids, biosynthesis and genetic regulation, Addison-Wesley, Reading, Mass.
3 - Umbarger, H. E. (1983), pp. 245-266. In Herrmman, K. M., Somerville, R. L. (Eds.); Amino acids, biosynthesis and genetic regulation, Addison-Wesley, Reading, Mass.
4 - Umbarger, H. E. (1987), pp. 353-367. In Neidhart, F. C., Ingraham, J. L., Low, K. B., Magasanik, B., Schaechter, M. and Umbarger, H. E. (Eds.); Escherichia coli and Salmonella typhimurium : Cellular and Molecular Biology, vol. 2, American Society for Microbiology, Washington, D. C.
5 - Piggot, P.G., and Hoch, J. A. (1985), Microbiological Reviews. 49 : 158-179.
6 - Pattee, P. A. (1976), J. Bacteriol. 127 : 1167-1172.
7 - Gibson, T. J., Ph. D thesis, University of Cambridge, Cambridge, England.
8 - Terzaghi, B. et Sandine, W. E. (1975), Appl. Microbiol. 29 : 807-813.
9 - Smid, E. J. et Konings, W. N. (1990), J.
   Baceteriol. 172 : 5286-5292.
10 - Maniatis, T., Fritsh, E. F., Sambrook, J. (1992) Molecular Cloning : a Laboratory Manual. Cold Spring Harbor Laboratory, Cold Spring Harbor, N. Y.
11 - Anagnostopoulos, C. et Spizizen, J. (1961), J. Bacteriol., 81 : 741-746.
10 - Corpet, F. 1988. Nucleic Acids Res. 16:10881-10890.
11 - Diderichsen, B., U. Wedsted, L. Hedegaard, B. R. Jensen, C. Sjoholm. 1990. J. Bacteriol. 172:4315-4321.
12a - Simon, D., and Chopin, A. (1988), Biochimie 70 : 559-566.
12b - Loureirodos Santos, A. L., Chopin, A. (1987), FEMS Microbiol. Lett. 42:209-212.
13 - Holo, H., Nes, I. F. (1991), Genetic and Molecular Biology of *Streptococci, Lactococci* and Enterococci, p. 298 (Ed. Dunny, G., Cleary, P, McKay, L.).
14 - Corpet, F. (1988), Nucleic Acids Res., 16 : 10881-10890.
15 - Van Rooyen, R. J. et De Vos, W. M. (1990), J. Biol. Chem. 265 : 18499-18503.
16 - Noirot, P., Petit, M. A., Ehrlich, D. S. (1987), J. Mol. Biol. 196 : 39-48.
17 - Diderichsen, B., Wedsted, U., Hedegaard, L., Jensen, B. R., Sjoholm, C. (1990) J. Bacteriol. 172 : 4315-4321.
18 - Sone, H., Fujii, T., Kondo, K., Shimizu, F., Tanaka, J., Inoue, T. (1988), Appl. Environm. Microbiol. 54 : 38-42.
19 - Halpern, Y. S.,Umbarger, H. E. (1959) J. Biol. Chem. 234 : 3067-3071.
20 - Holtzclaw, W. D.,Chapman, L. F. (1975) J. Bacteriol. 121 : 917-922.
21 - Zahler, S., Najimudin, N., Kessler, D., Vandeyar, M. (1988) : Biosynthesis of Branched Chain Amino Acids; pp. 26-42 (Ed. Barak, Z., Chipman, D. M., Schloss, F. U.)

## Revendications

1. Acide nucléique **caractérisé en ce qu'**il comprend la séquence d'ADN de la figure 2 ou un fragment de celle-ci de taille suffisante pour présenter l'activité α-acétolactate décarboxylase, et **en ce qu'**il code pour un polypeptide capable de transformer l'α-acétolactate en acétoine, et/ou pour un polypeptide capable d'activer une séquence d'ADN codant pour un polypeptide capable de transformer l'α-acétolactate en acétoine.

2. Acide nucléique selon la revendication 1, **caractérisé en ce qu'**il comprend la séquence d'ADN délimitée par les nucléotides situés aux positions 338 et 1045 de la figure 2 ou un fragment de celle-ci de taille suffisante pour présenter l'activité α-acétolactate décarboxylase, et **en ce qu'**il code pour un polypeptide capable de transformer l'α-acétolactate en acétoine.

3. Acide nucléique selon la revendication 1, **caractérisé en ce qu'**il comprend la séquence d'ADN délimitée par les nucléotides situés aux positions 1089 et 1466 de la figure 2 ou un fragment de celle-ci de taille suffisante pour présenter l'activité α-acétolactate décarboxylase, et **en ce qu'**il code pour un polypeptide capable d'activer une séquence d'ADN codant pour un polypeptide capable de transformer l'α-acétolactate en acétoine.

4. Acide nucléique, **caractérisé en ce qu'**il comprend une séquence d'ADN selon la revendication 2 codant pour un polypeptide capable de transformer l'α-acétolactate en acétoine, ainsi qu'une séquence d'ADN selon la revendication 3 codant pour un polypeptide activateur de l'expression de la séquence d'ADN codant pour le polypeptide capable de transformer l'α-acétolactate en acétoine.

5. Acide nucléique **caractérisé en ce qu'**il comprend une séquence nucléotidique selon l'une des revendications 1 à 4, associée avec un promoteur sous le contrôle duquel la transcription de ladite séquence est susceptible d'être effectuée et, le cas échéant, une séquence d'ADN codant pour un ou plusieurs signaux de terminaison de la transcription.

6. Vecteur recombinant capable de transformer une cellule hôte, ledit vecteur contenant un acide nucléique selon l'une quelconque des revendications 1 à 5 sous le contrôle d'élements de régulation permettant l'expression de cet acide nucléique dans la cellule hôte.

7. Procédé de préparation d'une levure capable de surproduire de l'acétoine, **caractérisé en ce que** la levure a été transformée avec un acide nucléique selon la revendication 5 ou un vecteur recombinant selon la revendication 6.

8. Levure capable de surproduire de l'acétoine, **caractérisé en ce qu'**elle a été transformée avec un acide nucléique selon l'une quelconque des revendications 1 à 5.

9. Procédé de préparation de bactérie du type L. lactis dont le gène aldB et/ou le gène aldR sont inactivés et donc capables de surproduire du diacétyle, **caractérisé en ce que** :
- l'on choisi deux segments d'au moins 300 pb situés soit, de part et d'autre ou dans l'acide nucléique selon la revendication 2 délimité par les nucléotides situés aux positions 338 et 1045, soit de part et d'autre ou dans l'acide nucléique selon la revendication 3 délimité par les nucléotides situés aux positions 1089 et 1466,
- l'on prépare un acide nucléique dont chaque extrémité est constituée de l'un des segments précédents,
- l'on introduit ledit acide nucléique dans un vecteur, lequel est à son tour introduit dans la souche hôte afin d'obtenir par une technique d'échange d'allèles ou de double recombinaison des batéries dont le gène aldB ou le gène aldR, sauvages, sont inactivés.

10. Procédé selon la revendication 9, **caractérisé en ce que** l'inactivation du gène aldB ou du gène aldR est réalisée par délétion de l'un ou l'autre de ces gènes au moyen d'un acide nucléique essentiellement constitué des segments d'au moins 300 pb.

11. Procédé selon la revendication 9, **caractérisé en ce que** l'inactivation du gène aldB ou du gène aldR est réalisée par remplacement de l'un ou l'autre de ces gènes par un gène muté, au moyen d'un acide nucléique délimité par les segments d'au moins 300 pb entre lesquels est disposé un gène muté.

12. Procédé selon l'une des revendications 9 à 11, **caractérisé en ce que** l'acide nucléique introduit dans un vecteur comporte également entre les segments de au moins 300 pb un marqueur permettant de sélectionner les bactéries dont le gène aldB est inactivé.

13. Procédé de préparation de bactérie du type *L. lactis* capable de surproduire du diacétyle, selon l'une quelconque des revendications 9 à 12, caratérisé en ce que ladite bactérie est en outre transformée par introduction directement dans son génome ou via un vecteur type plasmide, d'un gène codant pour une α-acétolactate synthase.

14. Bactérie type *L. lactis* capable de surproduire du diacétyle, préparée conformément au procédé selon l'une quelconque des revendications 9 à 13.

15. Polypeptide possédant une activité α-acétolactate décarboxylase, **caractérisé en ce qu'**il est constitué de tout ou partie de la séquence polypeptidique de la figure 2 codée par un acide nucléique selon la revendication 2.

16. Procédé de sélection de bactéries de type *L. lactis*, d'origine végétale ou laitière, présentant une déficience α-acétolactate décarboxylase et donc surproduisant du diacétyle, **caractérisé en ce qu'**il comprend les étapes suivantes :
- le traitement d'une bactérie, éventuellement préalablement sélectionnée sur un milieu de culture, de façon à rendre les acides nucliques qu'elle contient apte à s'hybrider avec une sonde,
- la mise en contact lesdits acides nucléiques avec une sonde ou mélange de sonde, éventuellement marquée, constituée de tout ou partie d'un acide nucléique selon l'une quelconque des revendications 1 à 5, dans des conditions permettant la formation de complexe d'hybridation entre la ou les sondes et les acides nucléiques provenant de la bactérie,
- la mise en évidence par tous moyens appropriés les hybrides éventuellement formés afin de déterminer la présence de mutation s'opposant à la formation desdits hybrides et indiquant une déficience α-acétolactate décarboxylase.

17. Procédé de culture de Lactococcus lactis permettant la sélection de mutants dépourvus d'activité α-acétolactate décarboxylase résistant à la Leucine, comprenant une étape de culture de Lactococcus lactis sur un milieu dépourvu d'isoleucine et de valine.

## Claims

1. Nucleic acid **characterized in that** it contains the DNA sequence of figure 2 or a fragment thereof, of sufficient size to have α-acetolactate decarboxylase activity, and **in that** it codes for a polypeptide able to convert the α-acetolactate into acetoin, and/or for a polypeptide able to activate a DNA sequence encoding a polypeptide able to convert α-acetolactate into acetoin.

2. Nucleic acid according to claim 1, **characterized in that** it contains the DNA sequence delimited by the nucleotides located at positions 338 and 1045 of figure 2, or a fragment thereof, of sufficient size to have α-acetolactate decarboxylase activity, and **in that** it encodes a polypeptide able to convert α-acetolactate into acetoin.

3. Nucleic acid according to claim 1, **characterized in that** it contains the DNA sequence delimited by the nucleotides located at positions 1089 and 1466 of figure 2, or a fragment thereof, of sufficient size to have α-acetolactate decarboxylase activity, and **in that** it encodes a polypeptide able to activate a DNA sequence coding for a polypeptide able to convert α-acetolactate into acetoin.

4. Nucleic acid, **characterized in that** it contains a DNA sequence according to claim 2 coding for a polypeptide able to convert α-acetolactate into acetoin, and a DNA sequence according to claim 3 coding for a polypeptide activating the expression of the DNA sequence encoding the polypeptide able to convert α-acetolactate into acetoin.

5. Nucleic acid **characterized in that** it contains a nucleotide sequence according to any of claims 1 to 4, associated with a promoter under whose control the transcription of said sequence may be made, and optionally, a DNA sequence coding for one or more transcription terminator signals.

6. Recombinant vector able to convert a host cell, said vector containing a nucleic acid according to any of claims 1 to 5 under the control of regulatory elements enabling the expression of this nucleic acid in the host cell.

7. Method for preparing a yeast able to overproduce acetoin, **characterized in that** the yeast was converted with a nucleic acid according to claim 5 or a recombinant vector according to claim 6.

8. Yeast able to overproduce acetoin, **characterized in that** it was converted with a nucleic acid according to any of claims 1 to 5.

9. Method for preparing bacteria of L.lactis type whose aldB gene and/or aldR gene are inactivated and therefore able to overproduce dicetyl, **characterized in that**:
- two segments of at least 300 pb are chosen and either positioned either side of or in the nucleic acid according to claim 2 delimited by the nucleotides located at positions 338 and 1045, or either side of or in the nucleic acid according to claim 3 delimited by the nucleotides located at positions 1089 and 1466,
- a nucleic acid is prepared of which each end is formed of one of the preceding segments,
- said nucleic acid is inserted in a vector, which in turn is inserted in the host strain in order to obtain bacteria, using an allele exchange or double recombination technique, whose wild aldB gene or aldR gene are inactivated.

10. Method according to claim 9, **characterized in that** the inactivation of the aldB gene or aldR gene is made by deleting either one of these genes by means of a nucleic acid essentially made up of the segments of at least 300 pb.

11. Method according to claim 9, **characterized in that** the inactivation of the aldB gene or aldR gene is made by replacing either one of these genes by a mutant gene, by means of a nucleic acid delimited by the segments of at least 300 pb between which a mutant gene is placed.

12. Method according to any of claims 9 to 11, **characterized in that** the nucleic acid inserted in a vector also comprises, between the segments of at least 300 pb, a marker with which it is possible to select the bacteria whose aldB gene is inactivated.

13. Method for preparing a bacterium of L. lactis type able to overproduce diacetyl, according to any of claims 9 to 12, **characterized in that** said bacterium is in addition converted by the insertion, directly into its genome or via a plasmid type vector, of a gene coding for a α-acetolactate synthase.

14. Bacterium of L. lactis type able to overproduce diacetyl, prepared in accordance with the method according to any of claims 9 to 13.

15. Polypeptide having α-acetolactate decarboxylase activity, **characterized in that** it is made up of all or part of the polypeptide sequence of figure 2 encoded by a nucleic acid according to claim 2.

16. Method for selecting bacteria of L. lactis type, of plant or dairy origin, having α-acetolactate decarboxylase deficiency and therefore being overproductive of diacetyl, **characterized in that** it comprises the following steps:
- treatment of a bacterium, optionally previously selected on a culture medium, so as to make the nucleic acids it contains able to hybridize with a probe,
- contacting said nucleic acids with a probe or probe mixture, optionally labelled, made up of all or part of a nucleic acid according to any of claims 1 to 5, under conditions enabling the formation of a hybridisation complex between the probe or probes and the nucleic acids derived from the bacterium,
- detection by any appropriate means of any hybrids formed in order to determine the presence of mutation opposing the formation of said hybrids and indicating α-acetolactate decarboxylase deficiency.

17. Culture method for Lactococcus lactis allowing the selection of leucine-resistant mutants free of α-acetolactate decarboxylase activity, comprising a culture step of Lactococcus lactis on a medium free of isoleucine and valine.

## Patentansprüche

1. Nukleinsäure, **dadurch gekennzeichnet, daß** sie die DNS-Sequenz aus Figur 2 enthält, oder ein Fragment derselben von hinreichender Größe um die α-Acetolactat-Decarboxylase-Aktivität aufzuweisen, und dadurch, daß sie für ein Polypeptid codiert, das α-Acetolactat in Acetoin umwandeln kann, und/oder für ein Polypeptid, das eine DNS-Sequenz aktivieren kann, die für ein Polypeptid codiert, das α-Acetolactat in Acetoin umwandeln kann.

2. Nucleinsäure nach Anspruch 1, **dadurch gekennzeichnet, daß** sie die DNS-Sequenz enthält, die durch die Nukleotide an den Positionen 338 und 1045 der Figur 2 begrenzt wird, oder ein Fragment dieser DNS-Sequenz von hinreichender Größe, um die α-Acetolactat-Decarboxylase-Aktivität aufzuweisen, und dadurch, daß sie für ein Polypeptid codiert, das α-Acetolactat in Acetoin umwandeln kann.

3. Nucleinsäure nach Anspruch 1, **dadurch gekennzeichnet, daß** sie die DNS-Sequenz enthält, die durch die Nukleotide an den Positionen 1089 und 1466 der Figur 2 begrenzt wird, oder ein Fragment dieser DNS-Sequenz von hinreichender Größe, um die α-Acetolactat-Decarboxylase-Aktivität aufzuweisen, und dadurch, daß sie für ein Polypeptid codiert, das eine DNS-Sequenz aktivieren kann, die für ein Polypeptid codiert, das α-Acetolactat in Acetoin umwandeln kann.

4. Nucleinsäure, **dadurch gekennzeichnet, daß** sie zum einen eine DNS-Sequenz nach Anspruch 2 enthält, die für ein Polypeptid codiert, das α-Acetolactat in Acetoin umwandeln kann, und zum anderen eine DNS-Sequenz nach Anspruch 3, die für ein Polypeptid codiert, das die Expression der DNS-Sequenz für das Polypeptid, das α-Acetolactat in Acetoin umwandeln kann, auslöst.

5. Nucleinsäure, **dadurch gekennzeichnet, daß** sie eine Nukleotidsequenz nach einem der Ansprüche 1 bis 4 enthält, die mit einem Promotor assoziiert ist, von dem die Transkription der DNS-Sequenz aktiviert wird, und gegebenenfalls Signale für den Abbruch der Transkription.

6. Rekombinanter Vektor, der zur Transformation der Wirtszelle fähig ist, wobei der Vektor eine Nukleinsäure nach irgendeinem der Ansprüche 1 bis 5 enthält, die von Mechanismen kontrolliert wird, die eine Expression dieser Nukleinsäure in der Wirtszelle erlauben.

7. Herstellungsverfahren für eine Hefe, die Acetoin im Überschuß produzieren kann, und das **dadurch gekennzeichnet** wird, daß die Hefe mit einer Nukleinsäure nach Anspruch 5 oder einem rekombinanten Vektor nach Anspruch 6 transformiert wurde.

8. Hefe, die Acetoin im Überschuß produzieren kann, und **dadurch gekennzeichnet, daß** sie durch eine Nukleinsäure nach irgendeinem der Ansprüche 1 bis 5 transformiert wurde.

9. Herstellungsverfahren für ein Bakterium des Typs L. lactis dessen Gen aldB und/oder dessen Gen aldR inaktiviert sind, und das also Diacetyl im Überschuß produzieren kann, und das **dadurch gekennzeichnet** wird, daß:
- man zwei Segmente von mindestens 300 Basenpaaren auswählt, die entweder auf beiden Seiten, oder innerhalb der Nukleinsäure nach Anspruch 2, begrenzt durch die Nukleotide an den Positionen 338 und 1045, liegen, oder aber auf beiden Seiten, oder innerhalb der Nukleinsäure nach Anspruch 3, begrenzt durch die Nukleotide an den Positionen 1089 und 1466
- man eine Nukleinsäure herstellt, bei der jedes Ende aus einem der vorerwähnten Segmente besteht
- man diese Nukleinsäure in einen Vektor einfügt, der dann in eine Wirtszelle eingebracht wird, um durch eine Technik des Allelaustausches oder der doppelten Rekombination Bakterien zu gewinnen, bei denen die Gene aldB oder aldR des Wildtyps inkaktiviert sind.

10. Verfahren nach Anspruch 9, das **dadurch gekennzeichnet** wird, daß die Inaktivierung des Gens aldB oder aldR durch Deletion des betreffenden Gens mittels einer Nukleinsäure, die im wesentlichen aus den Segmenten von mindestens 300 Basenpaaren besteht, durchgeführt wird.

11. Verfahren nach Anspruch 9, das **dadurch gekennzeichnet** wird, daß die Inaktivierung des Gens aldB oder aldR durch Ersatz des betreffenden Gens mittels einer Nukleinsäure durchgeführt wird, die aus den Segmenten von mindestens 300 Basenpaaren besteht, zwischen die ein mutiertes Gen plaziert ist.

12. Verfahren nach irgendeinem der Ansprüche 9 bis 11, das **dadurch gekennzeichnet** wird, daß die Nukleinsäure, die in einen Vektor eingebracht wird, zwischen den Segmenten von mindestens 300 Basenpaaren auch einen Marker enthält, der es erlaubt, die Bakterien zu selektieren, deren Gen aldB inaktiviert ist.

13. Herstellungsverfahren für ein Bakterium des Typs L. lactis nach irgendeinem der Ansprüche 9 bis 12, das Diacetyl im Überschuß produzieren kann, und das darüber hinaus **dadurch gekennzeichnet** wird, daß das betreffende Bakterium zusätzlich mit einem Gen modifiziert wird, das für eine α-Acetolactat-Synthease codiert, und zwar entweder durch direkten Einbau in das Genom, oder über einen Vektor vom Plasmidtyp.

14. Bakterium des Typs L. lactis das Diacetyl im Überschuß produzieren kann, und das mit einem Verfahren nach irgendeinem der Ansprüche 9 bis 13 hergestellt worden ist.

15. Polypeptid mit α-Acetolactat-Decarboxylase-Aktivität, das **dadurch gekennzeichnet** wird, daß es ganz oder teilweise aus der Polypeptidsequenz der Figur 2 besteht und durch eine Nukleinsäure nach Anspruch 2 codiert wird.

16. Selektionsverfahren für Bakterien des Typs L. lactis die aus pflanzlichem Material oder aus Milch oder Milchprodukten gewonnen wurden und einen α-Acetolactat-Decarboxylase-Mangel aufweisen, das **dadurch gekennzeichnet** wird, daß es die folgenden Schritte beinhaltet:
- ein eventuell vorher auf einem Nährmedium selektiertes Bakterium wird behandelt, so daß die in ihm enthaltenen Nukleinsäuren fähig werden, sich mit einer Sonde zu hybridisieren
- diese Nukleinsäuren werden mit einer Sonde oder einer Sondenmischung, die auch markiert sein kann, und die ganz oder teilweise aus einer Nukleinsäure nach irgendeinem der Ansprüche 1 bis 5 besteht in Kontakt gebracht, bei Bedingungen, die die Bildung von Hybridisierungs-Komplexen zwischen der oder den Sonden und den Nukleinsäuren der Bakterien erlauben
- die eventuell gebildeten Hybride werden mit allen geeigneten Mitteln wahrnehmbar gemacht, um die Anwesenheit einer Mutation, die die Bildung dieser Hybride verhindert und einen α-Acetolactat-Decarboxylase-Mangel anzeigt zu bestimmen.

17. Verfahren zur Kultur von Lactococcus lactis das die Auswahl von Mutanten ohne α-Acetolactat-Decarboxylase-Aktivität und mit Leucin-Resistenz erlaubt, und das einen Schritt der Kultur von Lactococcus lactis beinhaltet, bei dem auf einem Nährmedium ohne Isoleucin und Valin gezüchtet wird.
